Europäisches Patentamt

European Patent Office

Office européen des brevets

(11) Veröffentlichungsnummer: **0 118 877**
**B1**

# (12) EUROPÄISCHE PATENTSCHRIFT

(45) Veröffentlichungstag der Patentschrift:
**29.10.86**

(21) Anmeldenummer: **84102437.5**

(22) Anmeldetag: **07.03.84**

(51) Int. Cl.⁴: **C 07 C 69/16,** C 07 C 67/055,
C 07 C 67/54

(54) **Verfahren zur Herstellung von Diacyloxibutenen.**

(30) Priorität: **15.03.83 DE 3309168**

(43) Veröffentlichungstag der Anmeldung:
**19.09.84 Patentblatt 84/38**

(45) Bekanntmachung des Hinweises auf die Patenterteilung:
**29.10.86 Patentblatt 86/44**

(84) Benannte Vertragsstaaten:
**BE DE FR GB IT**

(56) Entgegenhaltungen:
**GB - A - 2 051 805**

(73) Patentinhaber: **BASF Aktiengesellschaft,
Carl-Bosch-Strasse 38, D-6700 Ludwigshafen (DE)**

(72) Erfinder: **Schnabel, Rolf, Dr., Frankenstrasse 22,
D-6707 Schifferstadt (DE)**
Erfinder: **Kissau, Gerd, Dr., Kiefernweg 1 A,
D-4400 Muenster (DE)**
Erfinder: **Weitz, Hans-Martin, Dr., Auf dem Koeppel 40,
D-6702 Bad Duerkheim (DE)**
Erfinder: **Fischer, Rolf, Dr., Bergstrasse 98,
D-6900 Heidelberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von 1,4-Diacyloxibutenen durch Umsetzung von 1,3-Butadienen mit Carbonsäuren und Sauerstoff an einem Katalysator.

Aus der DE-PS 22 17 452 ist bekannt, dass man 1,4-Diacyloxibutene (im folgenden «1,4-DABE» genannt) erhält, wenn man 1,3-Butadien mit Sauerstoff und einer Carbonsäure an einem festen Katalysator umsetzt, der Pd und mindestens eines der Elemente Sb, Bi, Te und Se enthält. Nach den Angaben der DE-OS 24 17 658 kann man diese Synthese aus Butadien, Sauerstoff und Essigsäure in der Gas- oder Flüssigphase auch an Katalysatoren durchführen, die Pt und mindestens ein Element der 5. und 6. Hauptgruppe enthalten. In der DE-OS 25 42 925 wird der Vorschlag gemacht, die Umsetzung der Ausgangsstoffe in einem Reaktor unter Vermeidung einer Gasphase durchzuführen. Schliesslich ist es aus der DE-OS 27 47 634 bekannt, dass man anstelle von Butadien einen Butadien enthaltenden $C_4$-Crackschnitt verwenden kann.

Für eine technische Nutzung dieser Verfahren müssen mehrere Voraussetzungen erfüllt sein. So darf z.B. die Produktivität des Katalysators, d.h. die pro Zeiteinheit und Katalysator- bzw. Edelmetallmengeneinheit produzierte Menge an 1,4-DABE, einen bestimmten Wert nicht unterschreiten, da sich sonst die Aufwendungen für den relativ teuren Katalysator nicht rentieren. Auch muss dieser Wert über einen hinreichend langen Zeitraum bestehen bleiben, um zeitraubende und kostspielige Katalysatorwechsel und Katalysatorregenerierungen zu vermeiden. Da neben 1,4-DABE stets auch das nicht erwünschte «3,4-DABE» gebildet wird, ist es ferner sehr wesentlich, dass die Selektivität der Synthese, d.h. der 1,4-DABE-Anteil im Verfahrensprodukt, einen bestimmten Wert nicht unterschreitet.

Da der Anteil an 3,4-DABE im Reaktionsprodukt beträchtlich ist und z.B. bei den in den japanischen Offenlegungsschriften 72 090/1973 und 140 406/1975 beschriebenen Verfahren mehr als 15 Gewichtsprozent beträgt, ist es zur Verbesserung der Ausbeute an 1,4-DABE erforderlich, das 3,4-DABE durch Isomerisierung in 1,4-DABE umzuwandeln. Ein entsprechendes Isomerisierungsverfahren wird z.B. in den japanischen Offenlegungsschriften 30 616/1972 und 1 26 611/1975 beschrieben. Durch die Isomerisierung erhöht sich zwangsläufig die Zahl der Verfahrensstufen. Weitere Nachteile ergeben sich dadurch, dass die Isomerisierung von 3,4-DABE einen verhältnismässig niedrigen Wirkungsgrad hat, weil 3,4-DABE als eine Vinylverbindung dazu neigt, unerwünschte Reaktionen einzugehen.

Man hat auch schon versucht, Katalysatoren mit einer erhöhten Selektivität bezüglich der Bildung von 1,4-DABE zu entwickeln. So wird in der DE-PS 27 16 000 ein Verfahren beschrieben, bei dem man einen Katalysator verwendet, der Pd und J enthält. Mit Katalysatoren dieser Art werden jedoch keine guten Standzeiten erzielt, so dass der Katalysator oft ausgewechselt werden muss. Auch ist bei iodhaltigen Katalysatorsystemen die Korrosionsgefahr gross.

Es wurde nun gefunden, dass man bei der Herstellung von 1,4-Diacyloxibutenen durch Umsetzung von 1,3-Butadienen mit Carbonsäuren und Sauerstoff in Gegenwart eines Katalysators in einem Reaktor die Bildung von 3,4-DABE vorteilhaft dadurch zurückdrängen kann, dass man aus dem Reaktionsgemisch das bei der Umsetzung als Nebenprodukt gebildete 3,4-Diacyloxibuten abtrennt und ganz oder teilweise in den Reaktor zurückleitet.

Als 1,3-Butadiene kommen ausser Butadien auch substituierte Butadiene, wie Isopren, 2,3-Dimethyl-1,3-butadien, 1,3-Pentadiene, wie Piperylen oder durch Acyloxigruppen substituierte 1,3-Butadiene, wie 1-Acetoxibutadien, 1-Acetoxi-2-methyl-1,3-butadien oder 1-Acetoxi-3-methyl-1,3-butadien in Betracht. Die genannten Butadiene können für sich oder als Mischungen, die z.B. auch noch andere Kohlenwasserstoffe, wie Monoolefine oder Paraffinkohlenwasserstoffe enthalten, eingesetzt werden. Solche Mischungen stehen z.B. als sogenannte $C_4$-Schnitte zur Verfügung.

Als Carbonsäuren kommen insbesondere niedermolekulare Carbonsäuren, z.B. Fettsäuren mit 1 bis 3 C-Atomen, wie Ameisensäure, Essigsäure oder Propionsäure in Betracht.

Als Katalysatoren verwendet man vorzugsweise Pd und/oder Pt enthaltende Katalysatoren, zweckmässig in Form von Trägerkatalysatoren, bei denen die aktiven Bestandteile auf dem Trägermaterial, wie Aktivkohle, $SiO_2$ oder $Al_2O_3$ aufgebracht sind. Die aktive Katalysatormasse kann neben Pd oder Pt noch andere Metalle, wie Te, Cu, Sb, Se oder Bi enthalten. Katalysatoren dieser Art werden z.B. in DE-PS 22 17 452, DE-OS 24 17 658, DE-OS 29 43 407 und EP 27 979 beschrieben.

Die Umsetzung zur Herstellung der 1,4-Acyloxibutene nimmt man auf an sich bekannte Weise in der Gas- oder Flüssigphase, z.B. bei Temperaturen von 80 bis 120 °C und Drücken von 1 bis 100 bar vor. Dabei setzt man von den Reaktanten z.B. die folgenden Mengen ein: 0,2 bis 10, vorzugsweise 1 bis 5 Mol 1,3 Butadien, 10 bis 100, vorzugsweise 20 bis 50 Mol Carbonsäure und 0,1 bis 5, vorzugsweise 0,5 bis 2,5 Mol Sauerstoff.

Nach dem erfindungsgemässen Verfahren wird aus dem Reaktionsgemisch, das bei der katalytischen Umsetzung der 1,3-Butadiene mit den Carbonsäuren und Sauerstoff erhalten wird, 3,4-DABE abgetrennt und ganz oder teilweise in den Reaktor zurückgeleitet. Die Abtrennung erfolgt zweckmässigerweise durch Destillation.

Überraschenderweise wird durch die erfindungsgemässe Rückführung von 3,4-DABE in die Reaktion die Neubildung von 3,4-DABE zurückgedrängt. Dieses Ergebnis konnte nicht erwartet werden, da keine Bedingungen vorliegen, die eine Isomerisierung von 3,4-DABE zu 1,4-DABE und umgekehrt zulassen, wodurch sich das thermodynamische Gleichgewicht zwischen den Isomeren (3,4-DABE:1,4-DABE= 1:3) einstellen könnte. Das ist daran zu erkennen, dass bei einer zusätzlichen 3,4-DABE-Zufuhr in den Reaktor über das thermodynamische Gleichgewicht hinaus keine Umwandlung von 3,4-DABE zu 1,4-DABE stattfindet, und weiterhin daran, dass beim Unterlassen der Rückführung das 3,4-DABE/1,4-DABE-Verhältnis – offenbar kinetisch kontrolliert – mit 1:10 weit unterhalb des thermodynamischen Gleichgewichts liegt. Weiterhin ist überraschend, dass bei maximaler Rückführung von 3,4-DABE, wodurch die Neubildung an 3,4-DABE praktisch vollständig zurückgedrängt wird, die 1,4-DABE-Synthese mit unverminderter Raum-Zeit-Ausbeute fortgesetzt werden kann, und zwar ohne Beeinträchtigung der Katalysatorstandzeit und der Selektivität (keine Erhöhung der Nebenproduktkonzentration).

Man verfährt im einzelnen so, dass man die Umsetzung der Ausgangsstoffe in dem Reaktor auf an sich übliche Weise entsprechend der Sumpf- oder Rieselfahrweise vornimmt, wobei man als Reaktor z.B. einen Festbett- oder Suspensionsreaktor verwendet. Der Sauerstoff wird gegebenenfalls mit Inertgasen verdünnt eingesetzt. Man kann den Sauerstoff auch in einem vorgeschalteten Sättiger in der Carbonsäure lösen, und diese beiden Reaktanten dann zusammen in den Reaktor einleiten. Zusätzlich wird das zweckmässigerweise durch Destillation aus dem flüssigen Reaktionsgemisch abgetrennte 3,4-DABE in den Reaktor geleitet. Bei Rückführung der gesamten aus dem Reaktionsgemisch abgetrennten 3,4-DABE-Menge wird die Neubildung an 3,4-DABE praktisch vollständig unterdrückt, d.h. die zurückgeführte Menge an 3,4-DABE ist praktisch gleich der Menge an ausgetragenem 3,4-DABE. Bei dieser Arbeitsweise stellt sich die für die vollständige Zurückdrängung der 3,4-DABE-Neubildung erforderliche 3,4-DABE-Konzentration nach dem Beginn der Umsetzung selbsttätig ein. Sie liegt dann konstant bei etwa 30% des Wertes für 1,4-DABE.

Man kann, je nach etwaigem Bedarf an 3,4-DABE, auch so verfahren, dass man das aus dem flüssigen Reaktionsgemisch abgetrennte 3,4-DABE nur zum Teil in den Reaktor zurückführt.

Die Aufarbeitung des Reaktionsgemisches sei am Beispiel der Umsetzung von 1,3-Butadien mit Essigsäure und Sauerstoff folgendermassen erläutert. Das Reaktionsgemisch, das noch gasförmigen oder gelösten Sauerstoff und gegebenenfalls Inertgase enthält, wird zur Rückgewinnung und Wiederverwendung dieser Gase nach dem Austragen aus dem Reaktor bei Temperaturen von 20 bis 40 °C auf Normaldruck oder leicht erhöhtem Druck entspannt. Dabei gehen der gelöste Sauerstoff und gegebenenfalls vorhandene Inertgase weitgehend sowie kleinere Anteile von Butadien und eventuell vorhandene andere Kohlenwasserstoffe in die Gasphase über. Der Butadienanteil kann aus der Gasphase durch bekannte Methoden, wie Wäsche, beispielsweise mit Essigsäure, oder durch Kältebehandlung entfernt werden. Sauerstoff und gegebenenfalls vorhandene Inertgase können dann dem Reaktor mittels eines Kompressors wieder zugeführt werden.

Das flüssige Reaktionsgemisch, das noch 1,3-Butadien und eventuell andere Kohlenwasserstoffe enthält, wird nun unter Normaldruck oder leicht erhöhtem Druck bis zum Sieden erhitzt, so daß Butadien und eventuell andere Kohlenwasserstoffe in die Gasphase übergehen, während Essigsäure sowie die anderen höher siedenden Anteile des Reaktoraustrags in der Flüssigphase bleiben. Diese Stofftrennung findet zweckmässigerweise in einer Destillationskolonne statt. Das gasförmige Butadien und eventuell vorhandene andere Kohlenwasserstoffe werden durch bekannte Methoden, z.B. durch Abkühlung und/oder Kompression verflüssigt und wieder dem Reaktor zugeführt, während man aus dem Reaktionsgemisch nicht umgesetzte Essigsäure, ebenfalls zum Zwecke der Wiederverwendung durch Destillation bei Normaldruck von den Diacetoxibutenen abtrennt.

Die schwersiedende Fraktion, die aus 1,4-Diacetoxibuten (1,4-DAcBE) und 3,4-Diacetoxibuten (3,4-DAcBE) besteht, wird nun zur Abtrennung des 3,4-DAcBE fraktioniert destilliert. Das geschieht zweckmässigerweise bei vermindertem Druck, damit Sumpf- und Kopftemperaturen in der Kolonne soweit abgesenkt werden, dass es nicht zu temperaturbedingten Zerfalls- oder Umwandlungsreaktionen kommt. Vorteilhaft sind Sumpftemperaturen zwischen 150 und 180 °C, was Drücke zwischen 60 und 80 mbar erfordert. Das dabei über Kopf abgetrennte 3,4-DAcBE wird erfindungsgemäss ganz oder teilweise in den Reaktor zurückgeführt.

Nach dem erfindungsgemässen Verfahren werden hohe Raum-Zeit-Ausbeuten und besonders hohe Selektivitäten erzielt, die sich über lange Zeiträume aufrechterhalten lassen.

Die nach dem Verfahren der Erfindung herstellbaren Diacyloxibutene sind wertvolle Zwischenprodukte, z.B. für die Herstellung von Butendiol-1,4, Tetrahydrofuran und Butandiol-1,4. Die bei der erfindungsgemässen Acetoxilierung von Isopren oder 1-Acetoxy -2- methyl-1,3-butadien erhaltenen 2-Methyl-1,4-diacetoxy -2- butene oder 1,1,4-Triacetoxy -2- methyl -2- butene sind wertvolle Zwischenprodukte, z.B. für die Synthese von Terpenverbindungen.

Beispiel
a) Herkömmliche Fahrweise
In ein Reaktionsrohr von 0,6 l Inhalt werden pro Stunde 1,25 l Essigsäure, in der 1,3 Mole Sauerstoff gelöst sind, und 0,6 l eines flüssigen Gemisches aus 1 Gewichtsteil 1,3-Butadien und 3 Gewichtsteilen Buten eingeleitet.

Das Reaktionsrohr ist gefüllt mit einem festen Trägerkatalysator, der aus SiO$_2$-Gel als Trägermaterial und den aktiven Komponenten Pd (3,4 Gew.-%), Te (0,8 Gew.-%) und Cu (10,5 Gew.-%) besteht. Der Katalysator hat eine Korngrösse von 1–3 mm.

Das Reaktionsrohr steht unter einem Druck von 70 bar. Die Temperatur wird auf 95 °C gehalten. Das flüssige Reaktionsgemisch wird zur Vermeidung von «hot spots» in einem Kreislauf mit 200 l/h umgepumpt. Dabei wird der zugeführte Sauerstoff in einem Sättiger, der in den Kreislauf integriert ist, in der Flüssigphase gelöst, so dass im eigentlichen Reaktionsraum keine Gasphase vorliegt.

Aus diesem Flüssigkeitskreislauf wird das Reaktionsgemisch in dem Masse entnommen, wie Ausgangsprodukte zugeführt werden. Das entnommene Reaktionsgemisch wird auf Normaldruck entspannt. Man erhält eine Flüssigphase, die neben der Hauptkomponente Essigsäure 2,1 Gew.-% 3,4-Diacetoxibuten-1 (3,4-DAcBE), 2,2 Gew.-% cis-1,4-Diacetoxibuten-2 (cis-1,4-DAcBE) und 18,4 Gew.-% trans-1,4-diacetoxibuten-2 (trans-1,4-DAcBE) enthält. Man destilliert aus dem Gemisch bei Temperaturen von 120 bis 140 °C und bei Drücken von 1 bar bis 130 mbar Essigsäure ab. Dann wird in einer 40-bödigen Rektifikationskolonne bei Temperaturen von 178 °C im Sumpf und 133 °C am Kopf bei 80 mbar 3,4-DAcBE von 1,4-DAcBE abdestilliert. 1,4-DAcBE wird schliesslich bei 13 mbar und 128 °C abdestilliert. Der Destillationsrückstand beträgt 0,2 Gew.-% bezogen auf die Flüssigphase nach Entspannen und Ausgasen.

b) Erfindungsgemässe Fahrweise

Dem Reaktionsrohr werden unter den im Absatz (a) genannten Bedingungen zusätzlich 132,5 g/h 3,4-DAcBE zugeführt, das durch die destillative Trennung des Gemisches von 1,4-DAcBE und 3,4-DAcBE erhalten wurde. Man entspannt das erhaltene Reaktionsgemisch wie beschrieben. Die erhaltene Flüssigphase enthält neben Essigsäure 10,6 Gew.-% 3,4-DAcBE, 2,2 Gew.-% cis-1,4-DAcBE und 18,5 Gew.-% trans-1,4-DAcBE. Die zugeführten und die ausgetragenen 3,4-DAcBE-Mengen sind praktisch identisch. Der Destillationsrückstand beträgt, wie bei der unter (a) beschriebenen Arbeitsweise ca. 0,2 Gew.-%. Nach einer 500-stündigen Versuchsdauer ist die Aktivität des Katalysators unverändert.

**Patentansprüche**

1. Verfahren zur Herstellung von 1,4-Diacyloxibutenen durch Umsetzung von 1,3-Butadienen mit Carbonsäuren und Sauerstoff in Gegenwart eines Katalysators in einem Reaktor bei Temperaturen von 80 bis 120 °C und Drücken von 1 bis 100 bar, dadurch gekennzeichnet, dass man aus dem Reaktionsgemisch das bei der Umsetzung als Nebenprodukt gebildete 3,4-Diacyloxibuten durch fraktionierte Destillation abtrennt und ganz oder teilweise in den Reaktor zurückleitet.

2. Verfahren nach Anspruch 1, dadurch gekennzeichnet, dass man die Abtrennung des 3,4-Diacyloxibutens aus dem Reaktionsgemisch durch Destillation vornimmt.

3. Verfahren nach Anspruch 1, dadurch gekennzeichnet, dass man von einem Reaktionsgemisch ausgeht, das durch Umsetzung von 1,3-Butadien mit Essigsäure und Sauerstoff in Gegenwart eines Palladium oder Platin enthaltenden Katalysators erhalten wurde.

**Revendications**

1. Procédé de préparation de 1,4-diacyloxybutènes par réaction de 1,3-butadiènes avec des acides carboxyliques et l'oxygène en présence d'un catalyseur, dans un réacteur à des températures de 80 à 120 °C et sous des pressions de 1 à 100 bars, caractérisé en ce qu'on sépare du mélange réactionnel, par distillation fractionnée, le 3,4-diacyloxybutène formé en tant que produit secondaire au cours de la réaction et on le renvoie en totalité ou en partie dans le réacteur.

2. Procédé selon la revendication 1, caractérisé en ce qu'on procède à la séparation du 3,4-diacyloxybutène d'avec le mélange réactionnel par distillation.

3. Procédé selon la revendication 1, caractérisé en ce qu'on part d'un mélange réactionnel qui a été obtenu par réaction de 1,3-butadiène avec l'acide acétique et l'oxygène en présence d'un catalyseur contenant du palladium ou du platine.

**Claims**

1. A process for the preparation of a 1,4-diacyloxybutene by reacting a 1,3-butadiene with a carboxylic acid and oxygen in the presence of a catalyst in a reactor at 80–120 °C and under 1–100 bars, wherein the 3,4-diacyloxybutene formed as by-product in the reaction is separated off from the reaction mixture by fractional distillation, and some or all of this 3,4-diacyloxybutene is recycled to the reactor.

2. A process as claimed in claim 1, wherein the separation of 3,4-diacyloxybutene from the reaction mixture is carried out by distillation.

3. A process as claimed in claim 1, wherein a reaction mixture obtained by reacting 1,3-butadiene with acetic acid and oxygen in the presence of a catalyst containing palladium or platinum is used as starting material.